(19) Europäisches Patentamt
European Patent Office
Office européen des brevets

(11) Veröffentlichungsnummer: **0 066 856**
**B2**

(12) # NEUE EUROPÄISCHE PATENTSCHRIFT

(45) Veröffentlichungstag der neuen Patentschrift: **17.11.88**

(51) Int. Cl.⁴: **C 07 C 101/22**, C 07 C 101/30

(21) Anmeldenummer: **82104892.3**

(22) Anmeldetag: **03.06.82**

(54) **Verfahren zur Herstellung von polyfunktionalen organischen Verbindungen mit wenigstens einer tert. Butyläther- oder -estergruppe.**

(30) Priorität: **05.06.81 DE 3122450**

(43) Veröffentlichungstag der Anmeldung:
**15.12.82 Patentblatt 82/50**

(45) Bekanntmachung des Hinweises auf die Patenterteilung:
**16.01.85 Patentblatt 85/3**

(45) Bekanntmachung des Hinweises auf die Entscheidung über den Einspruch:
**17.11.88 Patentblatt 88/46**

(84) Benannte Vertragsstaaten:
**AT BE CH DE FR GB IT LI LU NL SE**

(56) Entgegenhaltungen:
**DE - B - 1 593 939**
**GB - A - 1 374 327**

**J.Med.Chem. 9 (1966) 444**
**Liebigs Ann. Chem. 670 (1963), 127-136**
**Ind. eng. Chem. 28 (1936), 1187-8**
**Org. Syntheses 9 (1966) 444**
**J. Org. Chem. 28 (1963) 1251-1252**
**Weygand, Hilgetag, Organisch-Chemische Experimentierkunst, Leipzig 1970, p. 297**
**Chem. and Ind. 1959, 1121**
**Buehler, Pearson, Survey of Organic Syntheses, N.Y. 1970, p. 830**
**Organikum, 12. Auflage, Berlin 1973, pp. 155-6**

(73) Patentinhaber: **Birr, Christian, Dr., Prof., Werderstrasse 35, D-6900 Heidelberg (DE)**

(72) Erfinder: **Birr, Christian, Dr., Prof., Werderstrasse 35, D-6900 Heidelberg (DE)**

(74) Vertreter: **Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86 (DE)**



ACTORUM AG

**Beschreibung**

Die Erfindung betrifft ein Verfahren zur Herstellung von polyfunktionalen organischen Verbindungen mit wenigstens einer durch eine tert.-Butylgruppe selektiv blockierten funktionellen Gruppe mittlerer Nucleophilie.

Organische Verbindungen, die mehrere reaktive Gruppen enthalten, von denen nur eine gezielt in einem folgenden chemischen Reaktionsschritt umgesetzt werden soll, bedürfen einer selektiven Blockierung der nicht umzusetzenden Funktionen. Beispiele für Stoffklassen, in denen dieser Fall häufig auftritt, sind am Purin- oder Pyrimidingerüst derivatisierte Nucleinsäuren, sauer substituierte Zucker- und Aminozuckerderivate, Hydroxydicarbonsäuren, Aminodicarbonsäuren, Hydroxyaminosäuren und mehrfach sauer und/oder basisch substituierte Derivate von Dicarbonsäuren und Aminosäuren.

Es sind bereits zahlreiche Schutzgruppen bekannt, die sich für die Blockierung verschiedener funktioneller Gruppen eignen. Eine wesentliche Eigenschaft, die von derartigen Schutzgruppen gefordert ist, besteht in einer Abspaltbarkeit unter milden Bedingungen, welche andere Schutzgruppen oder reaktive Gruppen möglichst wenig beeinflusst.

Zu den Schutzgruppen, welche dieser Bedingung genügen, gehört die tert.-Butylgruppe, häufig als (tBu oder $Bu^t$) bezeichnet. Sie eignet sich beispielsweise zur Maskierung von Hydroxyl- und Carboxylgruppen. Dem Vorteil ihrer selektiven Spaltbarkeit unter schonenden Bedingungen steht jedoch der Nachteil eines sehr aufwendigen vielstufigen Verfahrens zur selektiven Einführung mit niedrigen Ausbeuten entgegen. Dieses Verfahren ist beispielsweise beschrieben in Houben-Weyl, «Methoden der organischen Chemie», vol. 15/1, S. 579 bis 584 und 649 bis 656, Georg Thieme Verlag, Stuttgart 1974. Die schwierige Zugänglichkeit von Verbindungen, welche diese Schutzgruppen tragen, stellt ein schwerwiegendes Handikap für deren breite Anwendung dar.

Aus GB-A Nr. 1374327 ist bereits ein Verfahren zur tert.-Butylierung organischer Verbindungen, die wenigstens eine freie Gruppe mittlerer Nucleophilie enthalten bekannt, bei dem man konzentrierte Phosphorsäure und Bortrifluorid in einer Lösung der organischen Verbindung in einem organischen Lösungsmittel auflöst und die erhaltene Lösung bei einer Temperatur von höchstens 0°C mit überschüssigem flüssigen Isobuten versetzt. Dieses Verfahren ist jedoch auf die Verwendung des äusserst umweltschädlichen Bortrifluorid angewiesen und bedarf daher besonderer Vorsichtsmassnahmen.

Der Erfindung liegt daher die Aufgabe zugrunde, diese Nachteile zu beseitigen und ein einfaches Verfahren zu schaffen, welches ohne komplizierte oder teure Vorrichtungen oder/und gefährliche Reagenzien zu erfordern, rasch in guter Ausbeute die Herstellung von polyfunktionalen Verbindungen gestattet, welche wenigstens eine durch eine tert.-Butylethergruppe selektiv blockierte funktionelle Funktion aufweisen.

Diese Aufgabe wird erfindungsgemäss gelöst durch ein Verfahren, welches dadurch gekennzeichnet ist, dass man eine entsprechende Verbindung mit wenigstens einer freien Gruppe mittlerer Nucleophilie in einer Lösung von konzentrierter Schwefelsäure in einem organischen Ether auflöst und die erhaltene Lösung bei einer Temperatur von höchstens 5°C drucklos mit überschüssigem flüssigem Isobuten umsetzt, die Schwefelsäure neutralisiert sobald der überwiegende Anteil der zu blockierenden Gruppe mit dem Isobuten reagiert hat und überschüssiges Isobuten abdampft.

Die üblichen, inorganischen Verbindungen vorkommenden funktionellen Gruppen weisen eine in der nachstehenden Reihe fallende Nucleophilie auf:

$H_2N$ - aliph. > Hs - aliph. > $H_2N$ - arom. > HO - aliph. > HS - arom. > HO - arom. > - COOH aliph ($\gamma$ > ß > $\alpha$) > - COOH arom. > - $OPO_3H_2$ > - $SO_2H$.

Gruppen mittlerer Nucleophilie sind in der obigen Reihe, wiederum nach abnehmender Nucleophilie angeordnet, die folgenden:

HO - aliph. > HS - arom. > HO - arom. > - COOH aliph. ($\gamma$ > ß > $\alpha$) > - COOH arom.

Die Gruppen dieser Reihe werden beim erfindungsgemässen Verfahren selektiv blockiert, wobei in der Regel die in dieser Reihe jeweils links stehende Gruppe überwiegend abreagiert, ehe eine in der Reihe weiter rechts stehende Gruppe in erheblichem Ausmass zu reagieren beginnt. Beispielsweise reagiert HO - aliph. in ca. 30 min., - COOH arom. in ca. 3 h. Unter den Bedingungen des Verfahrens der Erfindung werden die funktionellen Gruppen höchster Nucleophilie durch die Protonen der Schwefelsäure im Lösungsmittelgemisch blockiert. Gleichzeitig werden dabei die umzusetzenden Verbindungen im verwendeten Lösungsmittel in Lösung gebracht. Wenn die Lösung erfolgt ist, enthalten die umzusetzenden Verbindungen nur noch Funktionen mittlerer und niederer Nucleophilie. Wird nun erfindungsgemäss überschüssiges verflüssigtes Isobuten im angegebenen Temperaturbereich zugesetzt, so reagieren die Gruppen sehr niedriger Nucleophilie wie Phosphorsäuremonoester und Sulfonsäuregruppen gar nicht mehr mit dem Isobuten.

Für das erfindungsgemässe Verfahren können also polyfunktionalen organischen Verbindungen verwendet werden, welche wenigstens eine Hydroxylgruppe, Carboxylgruppe oder aromatische Sulfhydrylgruppe neben weiteren Gruppen mit höherer oder niedrigerer Nucleophilie aufweisen. Beispiele für derartige Verbindungen sind die weiter oben schon genannten Stoffklassen.

Der als Lösungsmittel verwendete organische

Ether wird so ausgewählt, dass die polyfunktionale organische Verbindung, an der die Blockierung durchgeführt werden soll, sich in Gegenwart von Schwefelsäure darin auflöst. Beispiele für geeignete Ether sind solche, die sich von Polyolen mit relativ kurzen organischen Ketten, worunter solche mit maximal 5 Kohlenstoffatomen verstanden werden, ableiten. Beispiele für geeignete Verbindungen sind die Ether von Glycolen und Polyäthylenglycolen wie Diethylenglycoldimethylether, Ether von Polyolen mit 3 bis 5 Hydroxylgruppen, beispielsweise Glycerintrimethylether, höhere Methoxyalkane, zyklische Ether, wie Dioxan und Tetrahydrofuran, Dimethoxyethan und ähnliche. Bevorzugt wird Dimethoxyethan.

Der Lösung von konzentrierter Schwefelsäure und der jeweiligen polyfunktionalen organischen Verbindung im ausgewählten Ether wird dann bei Temperaturen bis höchstens + 5, vorzugsweise etwa 0°C, überschüssiges flüssiges Isobuten zugesetzt und diese Temperatur, zweckmässig unter Rühren, aufrecht erhalten, bis die zu blockierende funktionelle Gruppe so weit reagiert hat, dass bereits die Blockierung mit einer weiteren Gruppe mit niedriger Nucleophilie aufzutreten beginnt. Normalerweise ist die Umsetzung einer Gruppe hierbei nicht quantitativ sondern wird nur bis zu einem gewissen Gleichgewichtszustand, bei dem die Redaktion in der Hauptsache abgelaufen ist, fortgeführt. Zu diesem Zeitpunkt enthält der Reaktionsansatz in der Regel immer noch etwas Ausgangsmaterial und zumeist auch kleinere Mengen weiterer Reaktionsprodukte. Diese lassen sich jedoch auf einfachste Weise, wie weiter unten beschrieben, abtrennen.

Sobald die Umsetzung weit genug fortgeschritten ist, wird die Reaktion abgebrochen durch rasche Entfernung des überschüssigen verbliebenen Isobutens und Neutralisation der Schwefelsäure. Die Neutralisation erfolgt zweckmässig durch Zugabe einer geeigneten Base, beispielsweise einem Alkalihydroxyd wie Natriumhydroxyd. Jedoch können auch andere ausreichend alkalisch reagierende Verbindungen verwendet werden. Das überschüssige Isobuten lässt sich leicht durch Abdampfen entfernen. Besonders günstig erfolgt das Abdampfen durch Verdunsten auf grosser Oberfläche. Nach Abtrennung von bei der Neutralisation der Schwefelsäure gegebenenfalls entstehenden unlöslichen Salzen, beispielsweise Natriumsulfat im Falle der Neutralisation mit Natronlauge, erfolgt die weitere Aufarbeitung und Reinigung zweckmässig durch Extraktion mit Wasser und chromatographische Auftrennung der wässerigen Phase. Vorzugsweise wird Etherlösungsmittel weitgehend abgezogen, beispielsweise durch Eindampfen in Vakuum. Der erhaltene Rückstand wird in Wasser aufgenommen und aus diesem die gewünschte Substanz durch Kristallisation gewonnen, oder die wässerige Lösung wird chromatographiert. Für die Chromatographie eignen sich besonders Molekularsiebmaterialien wie vernetzte Dextrane, beispielsweise die unter der Handelsbezeichnung Sephadex vertriebenen. Die Verbindungen der oben genannten Stoffklassen mit verschiedenen reaktiven Gruppen unterschiedlicher Nucleophilie sind auch nach partieller tert.-Butylierung wasserlöslich und können daher auf diese Weise leicht abgetrennt werden.

Andere geeignete Chromatographiematerialien sind z.B. Celluloeester und Ether wie Acetylcellulose und ähnliche Substanzen. Die Elution kann jeweils mit Wasser erfolgen, wobei die einzelnen Komponenten sich gut auftrennen lassen und rein erhalten werden.

Die Reaktionsdauer der Umsetzung mit Isobuten liegt im allgemeinen zwischen 10 und 120 min bei den Gruppen mit geringster Nucleophilie im Bereich der oben gegebenen Definition von mittlerer Nucleophilie können jedoch auch längere Reaktionszeiten auftreten. Für das Abbrechen der Reaktion durch Neutralisation und Entfernung von überschüssigem Reagens sind ebenfalls 10 bis 30 min zu rechnen, so dass man insgesamt ein äusserst rasches einfaches und ökonomisches Verfahren zur Verfügung hat, welches den Aufwand gegenüber älteren Verfahren drastisch herabsetzt. Bisher war es erforderlich, sich spezieller Schutzgruppen für alle nicht mit einer tert.-Butylgruppe zu schützenden Funktion zu bedienen, die dann später in einer Reihe zusätzlicher Schritte wieder abgespalten werden müssen, bevor die eigentliche tert.-Butylverbindung gewonnen werden kann. Unter den erfindungsgemässen Reaktionsbedingungen wird das Isobuten ohne Druckgefässe gehandhabt, was ebenfalls für die Praktikabilität des Verfahrens einen wesentlichen Vorteil darstellt.

Die folgenden Beispiele erläutern die Erfindung weiter:

Beispiel 1

Unter wasserfreien Bedingungen werden unter Rühren in 1 l 1,2-Dimethoxyethan 55 ml konz. Schwefelsäure gelöst. Darin werden 50 g L-Glutaminsäure eingetragen und gelöst. Das Gemisch wird anschliessend durch äussere Kühlung auf 0 bis +5°C abgekühlt und in einem Guss mit 150 ml verflüssigten Isobuten versetzt. Nach Rühren bei Eiskühlung wird die Reaktion nach 90 min abgebrochen, indem das Gemisch in eine Wanne gegossen wird. Hierin wird konz. Natronlauge eingegossen, äquivalent zu der eingesetzten Menge Schwefelsäure. Nach Umrühren wird das ausgeschiedene Natriumsulfat abgetrennt und das Filtrat im Vakuum eingedampft. Der sirupöse Rückstand wird in wenig Wasser gelöst und zur Abscheidung von Ausgangsmaterial mit 4 l Methanol versetzt, wobei Glutaminsäure ausfällt. Sie wird abfiltriert, das Filtrat in Vakuum eingedampft, der sirupöse Rückstand in wenig Wasser gelöst und auf eine Sephadex LH 20-Säule (∅ 10 bis 15 cm, Länge 1,5 m) aufgetragen. Die Säule wird mit Wasser eluiert, es tritt zuerst L-Glutaminsäure-α-tert.-butylester und dann als Hauptprodukt L-Glutaminsäure-γ-tert.-butylester aus. Ausbeute an L-Glutaminsäure-γ-tert.-butylester: 64%.

Statt durch chromatographische Reinigung

kann L-Glutaminsäure-y-tert.- butylester aus dem obengenannten sirupösen Rohprodukt auch durch Kristallisation aus Wasser gewonnen werden.

Beispiel 2

L-Asparaginsäure-ß-tert.-butylester

Es wurde wie im Beispiel 1 beschrieben, vorgegangen unter Verwendung von 1 l 1,2-Dimethoxyethan als Lösungsmittel und 120 ml konz. $H_2$-$SO_4$. In diese Lösung wurden 80 g L-Asparaginsäure eingebracht.

Die Isobutenmenge betrug 400 ml, die Reaktionszeit 60 min bis zum Abbruch. Die Ausbeute betrug 55% nach Isolierung des Produkts durch Kristallisation aus Wasser.

Beispiel 3

L-Threonin-tert.-butylether

Es wurde wieder wie in Beispiel 1 beschrieben, vorgegangen, jedoch wurden 56 ml konzentrierte Schwefelsäure, 42 g L-Threonin und 400 ml Isobuten eingesetzt. Die Reaktionszeit betrug 30 min. Ausbeute: 60%.

Vergleichsbeispiel

Synthese von Glutaminsäure-γ-tert.-butylester, über 7 Stufen, in Analogie zu bekannten Verfahren.

Das nachstehende Beispiel veranschaulicht die Herstellung der Verbindung von Beispiel 1 nach dem Stand der Technik.

1. N-Benzyloxycarbonylglutaminsäure (Z-Glu)

147 g (1 mol) L-Glutaminsäure werden in 2N NaOH gelöst und auf pH 9,5 eingestellt. Zu der Lösung werden 205 g Benzyloxycarbonylchlorid, verdünnt mit dem gleichen Volumen Dioxan, unter Rühren bei 20° C zugetropft. Der pH-Wert des Reaktionsgemisches wird hierbei mit Hilfe eines Autotitrators mit 4N NaOH auf pH 9,5 konstant gehalten, bis kein NaOH-Verbrauch mehr angezeigt wird (ca. 5 h). Zur Aufarbeitung wird die Reaktionslösung auf pH 11 gebracht, dreimal mit Ether ausgeschüttelt, um überschüssiges Benzyloxycarbonylchlorid zu entfernen, mit 2N HCl auf pH 2 angesäuert, mit NaCl gesättigt und dreimal mit Essigsäureethylester extrahiert. Die organische Phase wird sorgfältig abgetrennt, mit $Na_2SO_4$ getrocknet und im Vakuum bei 40°C eingedampft. Ausbeute 238 g (85%), Smp. 113 bis 114°C (aus Essigsäureethylester), RF-Wert in Benzol/Eisessig 7:1 0,30.

$C_{13}H_{15}NO_6$(281,27):

| | | | |
|---|---|---|---|
| Ber.: | C 55,51 | H 5,33 | N 4,98% |
| Gef.: | C 55,50 | H 5,55 | N 5,38% |

2. N-Benzyloxycarbonylglutaminsäureanhydrid

120,8 g (0,43 mol) Z-Glu werden in 200 ml wasserfreiem Tetrahydrofuran gelöst und bei 0°C mit der Lösung von 98 g Dicyclohexylcarbodiimid in 200 ml wasserfreiem Tetrahydrofuran versetzt. Nach 15 h Stehen bei – 20°C wird von ausgefallenem Dicyclohexylharnstoff abgesaugt, und das Lösungsmittel im Vakuum bei 30°C abgezogen. Das zurückbleibende Öl ist für die Weiterverarbeitung rein genug und wird sofort umgesetzt. Ausbeute 115 g (100%).

$C_{13}H_{15}NO_5$(263,25)

| | | | |
|---|---|---|---|
| Ber.: | C 59,31 | H 4,96 | N 5,32% |
| Gef.: | C 59,94 | H 5,59 | N 6,12% |

3. N-Benzyloxycarbonylglutaminsäure-a-ethylester

113 g (0,43 mol) Z-Gluanhydrid werden in 1 l wasserfreiem Äthanol gelöst und 15 h unter Rückfluss gekocht. Hierbei entstehen der α- und γ-Monoäthylester des Z-Glu nebeneinander im Verhältnis 1:2. Die Reaktionslösung wird im Vakuum eingedampft, der ölige Rückstand in 250 ml wasserfreiem Äther aufgenommen, mit 78 g destilliertem Dicyclohexylamin versetzt und 2 d im Kühlschrank stehengelassen. Das Kristallisat der α- und γ-Ester · Dicyclohexylammoniumsalze wird abgesaugt, und die Mutterlauge zur Rückgewinnung von Z-Glu im Vakuum eingedampft und mit 2N NaOH in Dioxan bei pH 10,5 am Autotitrator 5 h verseift. Das Dicyclohexylammoniumsalzgemisch wird zur Gewinnung des α-Esters in 300 ml Äthanol gelöst und mit ca. 1,5 l Benzin (40°C) zur Trübung versetzt. Zweckmässigerweise setzt man eine kleine Menge im voraus bereiteten α-Ester · Dicyclohexylammoniumsalzes als Impfkristalle zu. Das α-Ester · DCA-Salz kristallisiert bei 20°C in der Zeitspanne von 0,5 bis 4 h nahezu dc einheitlich aus und wird bis zur vollkommenen Reinheit noch zweimal aus Äthanol, dem im Verhältnis 1:5 zur Trübung Benzin (40°C) zugesetzt wird, umkristallisiert. Alle Mutterlaugen werden wie oben beschrieben zur Rückgewinnung von Z-Glu aufgearbeitet. Ausbeute an Z-Glu-α-äthylester · DCA-Salz 34 g (30%), Smp. 154°C (aus Äthanol/Benzin [40°C], $R_F$Wert in Benzol/Eisessig (7:1) 0,50.

$C_{27}H_{42}N_2O_6$ (490,65):

| | | | |
|---|---|---|---|
| Ber.: | C 66,25 | H 8,36 | N 5,72% |
| Gef.: | C 61,47 | H 7,72 | N 5,45% |

Z-Glu-γ-äthylester · DCA-Salz, Smp. 215°C (aus Äthanol/Benzin [40°C]), $R_F$-Wert in Benzol/Eisessig (7:1) 0,61.

4. 70 g des Z-Glu-a-äthylester · DCA-Salzes werden in 500 ml Essigsäureäthylester gelöst und dreimal mit je 100 ml 0,5N $KHSO_4$-Lösung extrahiert. Anschliessend wird die organische Phase dreimal mit 100 ml gesättigter NaCl-Lösung gewaschen, über $Na_2SO_4$ getrocknet und im Vakuum bei 30°C eingedampft. Ausbeute 48 g (100%), Smp. 46 bis 47°C (Äthanol/Benzin [40°C]), Drehwert $[a]_D^{19}$ (c = 2 in Methanol) –22,5° für Z-Glu-α-äthylester.

$C_{15}H_{19}NO_6$ (309,33):

| | | | |
|---|---|---|---|
| Ber.: | C 58,44 | H 5,84 | N 4,51% |
| Gef.: | C 58,00 | H 5,84 | N 4,71% |

Z-Glu-γ-äthylester, Smp. 230°C (Äthanol/Benzin [40° C]), Drehwert $[\alpha]_D^{19}$ (c = 2 in Methanol) -8°.

5. N-Benzyloxycarbonylglutaminsäure-a-äthyl-y-tert.-butylester

30 g Z-Glu-α-äthylester (0,1 mol) werden in 400 ml Dichlormethan gelöst und in einen Glasautoklaven gegeben. Unter Feuchtigkeitsausschluss werden 5 ml konz. Schwefelsäure und ca. 360 ml verflüssigtes Isobuten zugesetzt. In dem sorgfältig verschlossenen Reaktionsgefäss wird das Gemisch 4 d bei 20°C mit einem Magnetrührer gerührt. Vor dem Öffnen des Durchgefässes wird der Ansatz auf 0°C gekühlt und anschliessend in einen Kolben mit 200 ml 5% $Na_2CO_3$-Lösung überführt. Überschüssiges Isobuten wird mit einem Luftstrom in die Wasserstrahlpumpe gesaugt, und anschliessend die Dichlormethanphase abgetrennt. Die Sodalösung wird noch einmal mit Dichlormethan ausgeschüttelt, alle organischen Auszüge vereinigt, zweimal mit 5% Sodalösung und dreimal mit Wasser gewaschen, über $Na_2SO_4$ getrocknet und im Vakuum bei 30°C eingedampft. Das zurückbleibende Öl wird über $P_2O_5$ im Exsiccator getrocknet. Ausbeute 26 g (90%) Öl.

$C_{19}H_{27}NO_6$ (365,43):

| | | | |
|---|---|---|---|
| Ber.: | C 62,46 | H 7,39 | N 3,83% |
| Gef.: | C 63,35 | H 7,90 | N 3,30% |

6. N-Benzyloxycarbonylglutaminsäure-γ-tert.-butylesterdicyclohexylammoniumsalz

36,5 g (0,1 mol) Z-Glu (OBut) OEt werden in 300 ml Dioxan gelöst und am Autotitrator bei pH 10,5 mit 2N NaOH in 5 h bei 20°C unter kräftigem Rühren verseift. Zur Aufarbeitung wird das Reaktionsgemisch auf 0°C gekühlt, mit 1N HCl neutralisiert und im Vakuum zur Enfernung des Dioxans bei 40°C eingeengt. Der Rückstand wird mit 100 ml Wasser verdünnt, auf 0°C abgekühlt, mit 1N HCl auf pH 1,5 angesäuert und viermal mit je 100 ml Äther extrahiert. Der organische Auszug wird einmal mit 50 ml 5% $NaHCO_3$-Lösung und gesättigter NaCl-Lösung gewaschen, über $Na_2SO_4$ getrocknet und im Vakuum bei 30°C eingedampft. Das verbleibende Öl wird in 100 ml wasserfreiem Äther aufgenommen, mit 20 g dest. Dicyclohexylamin versetzt und 12 h im Eisschrank stehengelassen. Die ausgeschiedenen Kristalle werden abfiltriert, mit wenig wasserfreiem, kaltem Äther gewaschen und getrocknet. Ausbeute 48 g (95%), Smp. 133 bis 134°C (aus Äther); Drehwert $[a]_D^{19}$ + 6° (c = 2 in Methanol), $R_F$-Wert in Benzol/Eisessig (7:1) 0,32.

$C_{28}H_{45}N_2O_6$ (505,68):

| | | | |
|---|---|---|---|
| Ber.: | C 66,53 | H 8,91 | N 5,54% |
| Gef.: | C 67,24 | H 9,31 | N 5,61% |

7. L-Glutaminsäure-γ-tert.-butylester

43 g (0,85 mol) Z-Glu ($OBu^t$) · DCA werden in 300 ml Äther gelöst und zweimal mit 200 ml 0,5N $KHSO_4$-Lösung ausgeschüttelt. Die ätherische Phase wird zweimal mit gesättigter NaCl-Lösung gewaschen, über $Na_2SO_4$ getrocknet und im Vakuum bei 30°C eingedampft. Der Rückstand wird in 200 ml Methanol gelöst, mit ca. 2 g Palladium (10% auf Aktivkohle) versetzt und 7 h mit Hilfe eines Vibromischers in einem Wasserstoffstrom hydriert. Ein negativ ausfallender $CO_2$-Test zeigt das Ende der Schutzgruppenabspaltung an.

Der Katalysator wird abfiltriert, mit Methanol gespült, und das Filtrat im Vakuum bei 30°C eingedampft. Der Rückstand wird aus Methanol/wasserfreiem Äther umkristallisiert. Ausbeute 19 g (67%), Smp. 184°C (Methanol Äther). Der Katalysator wird abfiltriert, mit Methanol gespült, und das Filtrat im Vakuum bei 30°C eingedampft. Der Rückstand wird aus Methanol/wasserfreim Äther umkristallisiert. Ausbeute 19 g (67%), Smp. 184°C (Methanol Äther).

$C_9H_{17}N_1O_4$ (203,25):

| | | | |
|---|---|---|---|
| Ber.: | C 53,20 | H 8,37 | N 6,79% |
| Gef.: | C 52,52 | H 8,22 | N 6,58% |

**Patentansprüche**

1. Verfahren zur Herstellung von polyfunktionalen organischen Verbindungen mit wenigstens einer durch eine tert.-Butylgruppe selektiv blokkierten Gruppe mittlerer Nucleophilie neben weiteren Gruppen mit höherer oder niedrigerer Nucleophilie, dadurch gekennzeichnet, dass man eine entsprechende Verbindung mit wenigstens einer freien Gruppe mittlerer Nucleophilie in einer Lösung von konzentrierter Schwefelsäure in einem organischen Äther auflöst und die erhaltene Lösung bei einer Temperatur von höchstens 5°C drucklos mit überschüssigem flüssigem Isobuten umsetzt, die Schwefelsäure neutralisiert sobald der überwiegende Anteil der zu blockierenden Gruppe mit dem Isobuten reagiert hat und überschüssiges Isobuten abdampft.

2. Verfahren nach Anspruch 1, dadurch gekennzeichnet, dass das Abdampfen schnell, z.B. durch Verdunsten auf grosser Oberfläche erfolgt.

3. Verfahren nach einem der Ansprüche 1 bis 2, dadurch gekennzeichnet, dass man die neutralisierte Mischung mit Wasser extrahiert und die wässerige Phase über ein Molekularsiebmaterial chromatografiert oder das Produkt durch Kristallisation aus Wasser gewinnt.

**Claims**

1. Process for the preparation of polyfunctional organic compounds with at least one group of average nucleophilia selectively blocked by a tert.-butyl group, besides further groups with higher or lower nucleophilia, characterised in that one dissolves a corresponding compound with at least one free group of average nucleophilia in a solution of concentrated sulphuric acid in an organic ether and reacts the solution obtained with excess liquid isobutene at a temperature of at most 5°C., without pressure, neutralises the sulphuric acid as soon as the preponderant portion of the group to be blocked has reacted with the isobutene and evaporates off excess isobutene.

2. Process according to claim 1, characterised in that the evaporating off takes place quickly, e.g. by evaporation on a large surface area.

3. Process according to one of claims 1 to 2, characterised in that one extracts the neutralised mixture with water and chromatographs the aqueous phase over a molecular sieve material or obtains the product by crystallisation from water.

## Revendications

1. Procédé pour la préparation de composés organiques polyfonctionnels portant au moins un groupe à nucléophilie moyenne bloqué sélectivement par un groupe tert. butyle en plus d'autres groupes à nucléophilie supérieure ou inférieure, caractérisé en ce que l'on dissout un composé correspondant portant au moins un groupe libre à nucléophilie moyenne dans une solution d'acide sulfurique concentré dans un éther organique et l'on fait réagir la solution obtenue à une température de 5°C maximum sans pression avec un excès d'isobutène liquéfié, on neutralise l'acide sulfurique dès que la quantité prédominante du groupe à bloquer, a réagi avec l'isobutène et on élimine l'excès d'isobutène par évaporation.

2. Procédé selon la revendication 1, caractérisé en ce que l'on procède à une évaporation rapide, par exemple par vaporisation sur une grande surface.

3. Procédé selon l'une des revendications 1 et 2, caractérisé en ce que l'on extrait à l'eau le mélange neutralisé et on chromatographie la phase aqueuse sur une matière du type tamis moléculaire et on sépare le produit par cristallisation dans l'eau.